## Europäisches Patentamt
## European Patent Office
## Office européen des brevets

(19)

(11) Veröffentlichungsnummer: **0 355 149 B1**

(12)

# EUROPÄISCHE PATENTSCHRIFT

(45) Veröffentlichungstag der Patentschrift :
**11.12.91 Patentblatt 91/50**

(51) Int. Cl.⁵ : **A61K 35/78, A23F 3/34**

(21) Anmeldenummer : **89901737.0**

(22) Anmeldetag : **31.01.89**

(86) Internationale Anmeldenummer :
**PCT/HU89/00003**

(87) Internationale Veröffentlichungsnummer :
**WO 89/06966 10.08.89 Gazette 89/18**

(54) **EINE KRÄUTERTEE-KOMPOSITION UND VERFAHREN ZU DEREN HERSTELLUNG.**

(30) Priorität : **02.02.88 HU 43988**

(43) Veröffentlichungstag der Anmeldung :
**28.02.90 Patentblatt 90/09**

(45) Bekanntmachung des Hinweises auf die
Patenterteilung :
**11.12.91 Patentblatt 91/50**

(84) Benannte Vertragsstaaten :
**AT BE CH DE FR GB IT LI LU NL SE**

(56) Entgegenhaltungen :
**DE-A- 3 110 011**
**DE-B- 1 007 606**
**FR-A- 2 598 620**

(73) Patentinhaber : **EGAL Vegyipari Közös Vallalat**
**Bökényföldi u. 120**
**Budapest (HU)**

(72) Erfinder : **Csatordai, Jozsef**
**Dohany u. 20**
**H-7130 Tolna (HU)**
Erfinder : **Pámer, Frigyes**
**Bajcsy-Zs. u. 14/I**
**H-7130 Tolna (HU)**
Erfinder : **Orbán, Gyula**
**Alkotmány u. 3**
**H-7100 Szekszárd (HU)**
Erfinder : **Nagy, Lászlo**
**Pasaréti u. 67**
**H-1026 Budapest (HU)**

(74) Vertreter : **Hansen, Bernd, Dr.rer.nat. et al**
**Hoffmann, Eitle & Partner Patentanwälte**
**Arabellastrasse 4 Postfach 81 04 20**
**W-8000 München 81 (DE)**

## Beschreibung

Die Erfindung betrifft eine Kräutertee-Komposition und ein Verfahren zu deren Herstellung.

Es ist bekannt, daß ein Kräuterteegemisch mit sehr mannigfaltigem Wirkungsspektrum aus verschiedenen Heilpflanzen hergestellt werden kann. In den letzten Zeiten ist die Abneigung gegen die Anwendung synthetischer Verbindungen allgemein geworden; die Wissenschaft wendet sich mit lernewter Intensität dem Gebrauch von Wirkstoffen natürlichen Ursprungs zu.

Es ist auch bekannt, dass ein überraschend hohes Prozent der in modernen industriellen Gesellschaften lebenden Leute ein ungeordnetes Sexualleben hat und etwa 15 bis 20% der Männer mit sexuellen Potenzschwierigkeiten und ein bedeutender Teil der Franen mit Frigiditätsproblemen belastet sind.

Obwohl verschiedene synthetische Mittel und auch Hormonpräparate zur Lösung der obigen Probleme zur Verfügung stehen und die Psychotherapie sich in der letzten Zeitperiode stark entwickelt hat, vertritt der Erfinder die Meinning daß die Behandlung der sexuellen Störungen mit natürlichen Stoffen eine überaus wichtige Rolle in der ärztlichen Praxis spielen kann.

Ziel der Erfindung ist demnach die Zusammenstellung eines Kräuterteegemisches und daraus die Herstellung eines wirksamen heilkräftigen Getränkes, welches aus an sich bekannten, leicht zugänglichen und natürlichen Stoffen zusammengestellt werden kann.

In unseren Vorversuchen wurde es nun überraschenderweise gefunden, das, wenn man Hagedorn-Zweigesende mit Zitronelle mischt und einen Kräutertee aus dem derart gewonnenen Gemische bereitet, dessen ständiger Genuys einen sedativen-beruhigenden Effekt ausübt und gleichzeitig auch die Störungen des Sexuallebens vermindert.

Eine hervorragend günstige, die sexuelle Potenz und Bereitschaft erhöhende Wirkung wurde dann erreicht, wenn wie in dem Ansprüchen dargestellt, ein Gemisch von Wurmkrautblume, Hagedorn-Zweigensende und Zitronelle als Grundmaterialien des Teegemisches angewandt wurden.

Weiterhin wurde gefunden, das, wenn man einen Kräutertee nach Ergänzung des obigen Dreikomponentengemisches in dem entsprechenden Verhältnis mit an sich bekannten Roboriermitteln, Drogen, Stimulantien, Vitaminen mittels einer Brühungs- oder Tränkungsmethode bereitet, so die günstige Wirkung dieses Kräutertees eindeutig bewiesen werden kann.

Gegenstand der Erfindung ist demnach ein in dem Ansprüchen gekennzeichnetes Mittel und ein Verfahren zur Hertellung dieses Mittels, d.h. des die sexuelle Potenz und Bereitschaft erhöhenden Kräutertees und/oder heilkräftigen Getränkes durch die Anwendung an sich bekannter Drogen und Zusatzstoffe, indem man ein (getrocknetes) Gemisch von

10 bis 30 Gew.-%    Wurmkrautblume (Tanaceti flos),
10 bis 30 Gew.-%    Hagedorn-Zweigesende (Crataegi flos cum foliis),
5 bis 15 Gew.-%    Zitronelle (Melissa officinalis folium)

mit 25 bis 75 Gew.-% an sich bekanntem Roboriermittel, Droge, Stimulant, Vitamin ergänzt mit einer 2- bis 30-facher Menge Wassers bei Raumtemperatur tränkt und/oder brüht und gewünschtenfalls die zweierleien Filtrate vereinigt.

Gemäss einer bevorzugten Ausführungsform der Erfindung wird das Gemisch von

20 Gew.-%    Wurmkrautblume,
20 Gew.-%    Hagedorn-Zweigesende,
10 Gew.-%    Zitronelle, sowie
50 Gew.-%    anderem Roboriermittel, Droge, Stimulant, Vitamin zum Teekochen verwendet.

In unseren vergleichenden Versuchen erwies es sich am günstigsten das Gemisch von

20 Gew.-%    Wurmkrautblume,
20 Gew.-%    Hagedorn-Zweigesende,
10 Gew.-%    Zitronelle,
10 Gew.-%    Nussblatt,
8 Gew.-%    Preiselbeerenblatt,
10 Gew.-%    Kürbiskern,
5 Gew.-%    Mohrrübenwurzel,
10 Gew.-%    Weizenkeim,
5 Gew.-%    Citopan und
2 Gew.-%    Ascorbinsäure

zum Teekochen anzuwenden.

Im folgenden werden die in unseren Versuchen verwendeten Drogen mit der Darstellung ihrer bisher bekannten Wirkungen kurz erlänkt:

### Wurmkrautblume

Sie enthält 0,1 bis 0,3% ätherisches Öl (darin 70% Thujon), Bittersubstanz (Tonacetin), Metaarobinsäure und Harz.

Es ist bekannt, dass Thujon eine hypogastrische Hyperämie ausübt.

### Hagedorn

Er enthält Flavonoide, Terpene und Procyanidin, das eine Kräftige und dauerhafte Wirkung hat.

### Wirkungen:

Er hat einen sedativen-beruhigenden, positiv inotropen, koronargefässerweiternden Effekt, er erniedrigt den Blutdruck und, als ein kardiotonisches Agent, verbessert er den Koronardurchfluss des Herzens.

### Zitronelle

Sie enthält 0,1% ätherisches Öl, welches aus Aldehyden mit einer offenen Kohlenstoffkette (Citral, Citronellal) und Alkoholen (Geraniol, Linalool) besteht.

### Wirkungen:

Sie übt einen beruhigenden Effekt auf die Herzund Magennerven; sie ist ein Phytotranquillant (hebt die Erregung des sympathischen Zentrums des Hypothalamus auf).

### Nussblatt

Es enthält 0,01 bis 0,03% ätherisches Öl, Inosit, Gerbsäure, Juglandin, Juglon, Gallussäure, Naphthochinon, Gerbstoffe und Ascorbinsäure.

### Wirkungen:

Es übt einen blutreinigenden, magenverbessernden, verdauungsbefördernden, wurmtreibenden Effekt aus. Juglandin hemmt die Entwicklung der Pflanzenzellen.

### Liebstockelwurzel

Sie enthält 0,6 bis 1% ätherisches Öl (Terpineol, Phthalid), ausserdem Zucker, Stärke, Angelicinsäure, Karotin, Furocumarin und Umbellocumarin.

### Wirkungen:

Sie übt einen diuretischen und entzündungshemmenden Effekt aus

### Preiselbeerenblatt

Es enthält Arbutin, Vaccinin, Gerbstoffe, Gallusund Chinasäure.

### Wirkungen:

Es ist gegen die Entzündungen der Harnwege, des Nierenbeckens (Pyelitis) und der Harnblase (Cystitis) wirksam.

### Kürbiskern

Er enthält 20 bis 25% Öl, darin viele ungesättigte Fettsäuren; ausserdem hat er einen hohen Gehalt an Vitamin E.

Anwendung:

Im Falle von Blasenkatarrh und Prostataentzündung.

Der Vorteil der Erfindung besteht darin, dass in dieser Weise ein Teepräparat natürlichen Ursprungs mit stimulierender Wirkung hergestellt werden kann, das die sexuellen Störungen günstig beeinflusst. Wenn die angegebenen Dosenwerte eingehalten werden, so verursacht der verzehrte Tee bzw. Getränk keine schädigende Wirkung und beide können ohne irgendeine Kontraindikation verzehrt werden.

Das erfindungsgemässe Verfahren wird im folgenden an Hand von Ausführungsbeispielen näher erläutert.

Beispiel 1

a.) Zusammensetzung eines Teegemisches

20 g    Wurmkrautblume
20 g    Hagedorn-Zweigesende
10 g    Zitronelle
20 g    Weizenkeim
30 g    Nussblatt

b.) Bereitung und Verzehrung des Tees

2,5 dl Aufguss werden aus 20 g Teegemisch cerart bereitet, dass 4 gupfvolle Esslöffel des auszuziehenden Teematerials in Portionen zu 2,5 dl siedendem wasser gegeben werden, wonach der Topf bedeckt und sogleich von dem Feuer abgezogen wird. Man lässt den Aufguss 1 bis 1,5 Stunden lang abkühlen und dann wird er filtriert.

Es ist empfehlenswert, den Tee auch derart zu bereiten, dass eine Hälfte des gewonnenen Teematerials 8 Stunden lang in 2 dl kaltem Wasser getränkt wird und die andere Hälfte der täglichen Dosis mit 3 dl Wasser gebrüht und dann abgekühlt wird.

Vor dem Gebrauch werden beide Hälften beigemischt und filtriert.

Es ist zweckmässig den Tee kalt zu verzehren und gewünschterweise durch Honig zu versüssen. Die tägliche Dcsis beträgt 2,5 dl. Weiterhin ist es zweckmässig, die tägliche Dosis in drei Portionen allmählich zu verzehren, um die gleichmässige Absorption und das Konzentrations-niveau in den inneren Organen und im Blut zu versichern.

Eine Kur dauert 5 bis 7 Tage; darauffolgend ist es zweckdienlich 14 bis 20 Tage auszulassen.

Beispiel 2

a. Zusammensetzung eines Teegemisches

20 g    Wurmkrautblume
20 g    Hagedorn-Zweigesende
10 g    Zitronelle
10 g    Nussblatt
10 g    Kürbiskern
8 g     Preiselbeerenblatt
5 g     Mohrrübenwurzel
10 g    Weizenkeim
5 g     Zitopan
2 g     Ascorbinsäure

b.) Bereitung und Verzehrung des Tees

Man geht, wie im Beispiel 1 b) beschrieben, vor.

Beispiel 3

Die im Beispiel 2 a.) angegebenen Mengen der Drogen werden in einem zehnfachen Gewicht von Wasser aufgekocht, und der derart erhaltene wässrige Auszug wird filtriert. In 0,5 l des wässrigen Auszuges werden

EP 0 355 149 B1

0,1 kg Karamel, 3 g Saccharin und 0,02 kg Ascorbinsäure gelöst. Dann wird die Lösung mit Äthylalkohol in einem Verhältnis von 1:1 gemischt und ein Anis- und/oder Zitronen-Würzenextrakt wird (werden) zugegeben. Zubereitung: in Flaschen von 100 ml.

## Patentansprüche

1. Kräutertee-Komposition, **dadurch gekennzeichnet**, dass das Gemisch

| | |
|---|---|
| 10 bis 30 Gew.-% | Wurmkrautblume (Tanaceti flos), |
| 10 bis 30 Gew.-% | Hagedorn-Zweigesende (Crataegi flos cum foliis), |
| 5 bis 15 Gew.-% | Zitronelle (Melissa officinalis folium) |

und 25 bis 75 Gew.-% Roboriermittel, Droge, Stimulant und Vitamin enthält.

2. Verfahren zur Herstellung eines die sexuelle Potenz erhöhenden Kräutertees nach Anspruch 1, **dadurch gekennzeichnet**, dass man ein (getrocknetes) Gemisch von

| | |
|---|---|
| 10 bis 30 Gew.-% | Wurmkrautblume (Tanaceti flos), |
| 10 bis 30 Gew.-% | Hagedorn-Zweigesende (Crataegi flos cum foliis), |
| 5 bis 15 Gew.-% | Zitronelle (Melissa officinalis folium) |

mit 25 bis 75 Gew.-% an sich bekanntem Roboriermittel, Droge, Stimulant, Vitamin ergänzt in einer 2- bis 30-fachen Menge von Wasser bei Raumtemperatur tränkt und/oder brüht und gewünschtenfalls die zweierleien Filtrate vereinigt oder mit Äthylalkohol ergänzt.

3. Verfahren nach Anspruch 2, **dadurch gekennzeichnet**, dass man das Gemisch von

| | |
|---|---|
| 20 Gew.-% | Wurmkrautblume, |
| 20 Gew.-% | Hagedorn-Zweigesende, |
| 10 Gew.-% | Zitronelle, sowie |
| 50 Gew.-% | anderem Roboriermittel, Droge, Stimulant, Vitamin zum Teekochen verwendet. |

4. Verfahren nach Anspruch 2 oder 3, **dadurch gekennzeichnet**, dass man das Gemisch von

| | |
|---|---|
| 20 Gew.-% | Wurmkrautblume, |
| 20 Gew.-% | Hagedorn-Zweigesende, |
| 10 Gew.-% | Zitronelle, |
| 10 gew.-% | Nussblatt, |
| 8 Gew.-% | Preiselbeerenblatt, |
| 10 Gew.-% | Kürbiskern, |
| 5 Gew.-% | Mohrrübenwurzel, |
| 10 Gew.-% | Weizenkeim, |
| 5 Gew.-% | Citopan und |
| 2 Gew.-% | Ascorbinsäure zum Teekochen verwendet und die derart erhaltene Abkochung in einem Verhältnis von 1:1 mit Äthylalkohol ergänzt. |

## Claims

1. Herbal tea composition, characterised in that the mixture contains

| | |
|---|---|
| 10 to 30% wt. | tansy flowers (Tanaceti flos), |
| 10 to 30% wt. | hawthorn twig tips (Crataegi flos cum foliis), |
| 5 to 15% wt. | citronella (Melissa officinalis foluim) and |
| 25 to 75% wt. | roborants, drugs, stimulant and vitamins. |

2. Process for the production of a herbal tea that increases sexual potency according to Claim 1, characterised in that a (dried) mixture of

| | |
|---|---|
| 10 to 30% of | tansy flowers (Tanaceti flos), |
| 10 to 30% wt. of | hawthorn twig tips (Crataegi flos cum foliis), |
| 5 to 15% wt. | citronella (Melissa officinalis folium) supplemented |

with 25 to 75% of wt. of roborants known **per se**, drugs, stimulants, vitamins is soaked in 2 to 30 times the quantity of water at room temperature and/or brewed and if desired the two kinds of filtrate are united or supplemented with ethyl alcohol.

3. Process according to Claim 2, characterised in that a mixture of

| | |
|---|---|
| 20% wt. | tansy flowers, |
| 20% wt. | hawthorn twig tips, |
| 10% wt. | citronella and |

5

50% wt. other roborants, drugs, stimulants, vitamins, is used for making tea.

4. Process according to Claim 2 or 3, characterised in that a mixture of

20% wt. tansy flowers,
20% wt. hawthorn twig tips,
10% wt. citronella,
10% wt. walnut leaf,
8% wt. cranberry leaf,
10% wt. pumpkin seed,
5% wt. carrot root,
10% wt. cytopan, and
2% wt. ascorbic acid

is used for preparing the tea and the concoction thus obtained is supplemented in a ratio of 1 : 1 with ethyl alcohol.

**Revendications**

1. Composition pour tisane, caractérisée par le fait que le mélange contient:

10 à 30% en poids de fleurs de tanaisie (Tanaceti flos),
10 à 30% en poids de pousses d'aubépine (crataegi flos cum foliis),
5 à 15% en poids de citronnelle (melissa officinalis folium),
et 25 à 75% en poids de produit roboratif, médicament, stimulant et vitamine.

2. Procédé pour la fabrication d'une tisane selon la revendication 1, qui accroît la puissance sexuelle, caractérisée en ce que l'on fait macérer et/ou décocter à température ambiante dans une quantité d'eau 2 à 30 fois supérieure un mélange (sec) de:

10 à 30% en poids de fleurs de tanaisie (Tanaceti flos),
10 à 30% en poids de pousses d'aubépine (crataegi flos cum foliis),
5 à 15% en poids de citronnelle (melissa officinalis folium),
additionnés de 25 à 75% de produits roboratifs connus en soi, de médicament, de stimulant, de vitamine, et que, si on le souhaite, les filtrats des deux sortes sont mélangés ou complétés par de l'alcool éthylique.

3. Procédé selon la revendication 2, caractérisé en ce que l'on utilise le mélange de:

20% en poids de fleurs de tanaisie,
20% en poids de pousses d'aubépine,
10% en poids de citronnelle,
ainsi que 50% d'autres produits roboratifs, médicament, stimulant et de vitamine.

4. Procédé selon la revendication 2 ou 3, caractérisé en ce que l'on utilise pour la décoction de la tisane un mélange de:

20% en poids de fleurs de tanaisie,
20% en poids de pousses d'aubépine,
10% en poids de citronnelle,
10% en poids de feuilles de noix,
8% en poids de feuilles d'airelle rouge,
10% en poids de pépin de citrouille,
5% en poids de racines de carotte sauvage,
10% en poids de germes de blé,
5% en poids de citopan,
et 2% en poids d'acide ascorbique et que la décoction ainsi obtenue est complétée par de l'alcool éthylique dans le rapport 1/1.